# EUROPEAN PATENT SPECIFICATION

(11) **EP 3 897 393 B1**
(45) Date of publication and mention of the grant of the patent: **19.08.2026**
(21) Application number: 19829536.2
(22) Date of filing: 19.12.2019
(51) Int. Cl.: A61B 8/00, A61B 8/06, A61B 8/08

(54) **SYSTEM FOR MONITORING A FUNCTION OF A HEART**
SYSTEM ZUR ÜBERWACHUNG EINER HERZFUNKTION
SYSTÈME DE SURVEILLANCE D'UNE FONCTION CARDIAQUE

(30) Priority: 20.12.2018 US 201862782405 P
(43) Date of publication of application: 27.10.2021
(73) Proprietor: Koninklijke Philips N.V., 5656 AE Eindhoven (NL)
(72) Inventor: LAU, Kevin, Daniel, Seng, Hung, 5656 AE Eindhoven (NL); BARAGONA, Marco, 5656 AE Eindhoven (NL); MAESSEN, Ralph, Theodorus, Hubertus, 5656 AE Eindhoven (NL); PRATER, David, 5656 AE Eindhoven (NL)
(74) Representative: Philips Intellectual Property & Standards
(86) International application number: PCT/EP2019/086139
(87) International publication number: WO 2020/127615

(56) References cited:
- WO-A2-2004/002305
- US-A1- 2013 245 441
- VEJDANI-JAHROMI MARYAM ET AL: "Assessment of Diastolic Function Using Ultrasound Elastography", ULTRASOUND IN MEDICINE AND BIOLOGY, NEW YORK, NY, US, vol. 44, no. 3, 10 January 2018 (2018-01-10), pages 551 - 561, XP085413979, ISSN: 0301-5629, DOI: 10.1016/J.ULTRASMEDBIO.2017.11.011

## Description

### FIELD OF THE INVENTION

The invention relates to the field of non-invasive monitoring of a heart, and more specifically to the field of ultrasound heart monitoring.

### BACKGROUND OF THE INVENTION

The pumping function of the heart can be characterized by systolic ejection and diastolic filling. During ejection the heart contracts and actively stiffens, ejecting blood into the arterial circulation. Conversely during filling the heart relaxes back towards its passive stiffness, enabling the refilling of blood from the pulmonary circulation.

The ability to rapidly transition from a contractile state to a relaxed state enables a healthy heart to refill at low ventricular pressures. In cases of heart failure this ability to relax and/or the passive stiffness become impaired, resulting in abnormally elevated filling pressures.

The end-diastolic pressure-volume relationship (EDPVR) provides an approach to assess the passive stiffness of a ventricle. The EDPVR describes the non-linear relationship between pressure and volume at the end of filling as a function of volume. The passive stiffness of the ventricle can be estimated from the slope of the EDPVR at its current volume, a metric which has been linked to diastolic dysfunction as described in S. F. Nagueh et al., "Recommendations for the evaluation of left ventricular diastolic function by echocardiography," Eur. J. Echocardiogr., vol. 10, no. 2, pp. 165-193, 2009.

Typically, the EDPVR is determined by simultaneously measuring pressure and volume over a range of heartbeats. However, the measurement of ventricular pressure is only possible by invasive catheterization. The requirement of invasive catheters limits the measurement of EDPVR clinically.

There is therefore a need for a means of non-invasively determining the EDPVR.

US 2013/245441 (D1) discloses a method for determining pressure-volume information using ultrasound imaging.

### SUMMARY OF THE INVENTION

The invention is defined by the claims.

In particular, there is provided a computer program comprising computer program code means which is adapted, when said computer program is run on a computer, to implement a method for calculating a non-invasive end-diastolic pressure-volume relationship, as defined in claim 1.

Furthermore, there is provided a processing unit for calculating an end-diastolic pressure-volume relationship according to claim 11.

### SUMMARY OF THE DISCLOSURE

In an example, the calculating of the end-diastolic pressure-volume relationship comprises:
estimating an end-diastolic pressure at the end-diastolic volume of the left ventricle based on the linearized ventricular pressure-volume relationship; and
matching the estimated end-diastolic pressure to a generalized pressure-volume relationship, wherein the generalized pressure-volume relationship is derived from experimental measurements.

In this way, the estimated end-diastolic pressure may be used to link a given subject with experimental data. The experimental data may be obtained from a database and may include a wide range of data.

In an example, the determining of the end of diastasis volume of the left ventricle comprises generating a volume waveform of the left ventricle volume by performing an analytical integration of an aortic flow waveform and a mitral flow waveform.

In an arrangement, the fitting of the volume waveform to the segmentation of the left ventricle comprises performing a least-squares fitting.

In an example, the method comprises determining a number of heartbeats represented in the cardiac input.

If the number of heartbeats is greater than one, the generation of the linearized ventricular pressure-volume relationship comprises fitting an intercept to the linearized ventricular pressure-volume relationship.

When a plurality of heartbeats is available in the cardiac input, the intercept of the linearized ventricular pressure-volume relationship may be fit based on the data itself.

**In** a further example, if the number of heartbeats is one, the generation of the linearized ventricular pressure-volume relationship comprises fitting a constant intercept to the linearized ventricular pressure-volume relationship.

Where a single heartbeat is available, the intercept of the linearized ventricular pressure-volume relationship may be set to zero, or any constant value, thereby eliminating potentially erroneous intercepts being determined based on a single data point.

**In** an alternate example, if the number of heartbeats is one, the generation of the linearized ventricular pressure-volume relationship comprises estimating a non-zero intercept to the linearized ventricular pressure-volume relationship.

The intercept may be estimated based on a number of different data sources, such as historical patient data and/or data from patients having a similar condition.

**In** an arrangement, if the number of heartbeats is one, the calculating of the end-diastolic pressure-volume relationship comprises fitting the end-diastolic pressure-volume relationship based on a single heartbeat.

**In** an example, if the number of heartbeats is greater than one, the calculating of the end-diastolic pressure-volume relationship comprises performing a least-squares fitting of the end-diastolic pressure-volume relationship based on a plurality of heartbeats.

**In** an example, the method further comprises:
determining a gradient of the end-diastolic pressure-volume relationship at an end diastolic volume; and
if the gradient is greater than a predetermined threshold, generating an alert.

**In** an embodiment, the cardiac input comprises ultrasound data.

For example, the ultrasound data may include ultrasound image data, such as B-mode ultrasound data, and/or Doppler color ultrasound data.
Therefore, the alert can be generated either by an ultrasound system acquiring ultrasound data or a separate monitoring system.

**In** an example, the cardiac input comprises a cardiac model.

For example, the cardiac model may be a multi-scale model which represents the non-linear pressure-volume behavior of the heart.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 shows an ultrasound diagnostic imaging system to explain the general operation;
Figure 2 shows a method implemented by the computer program of the invention;
Figure 3 shows example plots of pressure-volume loops, highlighting the end-diastolic pressure-volume relationship (EDPVR);
Figure 4 shows a graph of the analytical flow waveforms against time as computed from the fitting of the analytical volume function to the segmented volumes of the subject;
Figure 5 shows a graph of volume against time for a left ventricle of a heart of the subject;
Figure 6 shows the graph of Figure 5 with a volume indicator positioned at the end of diastasis; and
Figure 7 shows a graph of pressure against volume for a left ventricle of a subject.

### DETAILED DESCRIPTION OF THE EMBODIMENTS

The invention will be described with reference to the Figures.

It should be understood that the detailed description and specific examples, while indicating exemplary embodiments, are intended for purposes of illustration only and are not intended to limit the scope of the invention. These and other features, aspects, and advantages of the embodiments of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The disclosure provides a computer program which implements a method for calculating an end-diastolic pressure-volume relationship. The method includes obtaining a cardiac input representing a region of interest, wherein the region of interest comprises a left ventricle and a left atrium of a subject. An end of diastasis volume of the left ventricle is then determined based on the cardiac input, wherein diastasis is a stage of diastole during a heart cycle before atrial contraction. Further, an end of diastasis pressure in the left atrium is determined based on the cardiac input and a linearized ventricular pressure-volume relationship is generated based on the end of diastasis volume of the left ventricle and the end of diastasis pressure in the left atrium . An end-diastolic pressure-volume relationship is then determined based on an end-diastolic volume of the left ventricle and the linearized ventricular pressure-volume relationship.

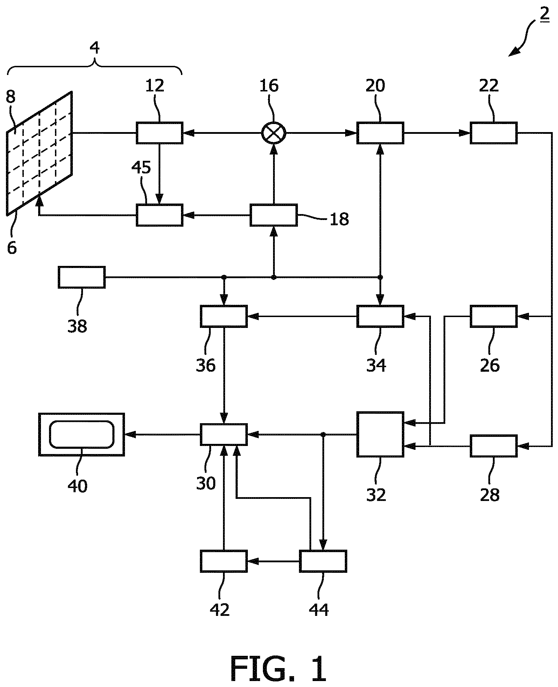

The general operation of an exemplary ultrasound system will first be described, with reference to Figure 1, and with emphasis on the signal processing function of the system since this invention relates to the processing of the signals measured by the transducer array.

The system comprises an array transducer probe 4 which has a transducer array 6 for transmitting ultrasound waves and receiving echo information. The transducer array 6 may comprise CMUT transducers; piezoelectric transducers, formed of materials such as PZT or PVDF; or any other suitable transducer technology. **In** this example, the transducer array 6 is a two-dimensional array of transducers 8 capable of scanning either a 2D plane or a three dimensional volume of a region of interest. **In** another example, the transducer array may be a 1D array.

The transducer array 6 is coupled to a microbeamformer 12 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 16, which the microbeamformer 12 can be coupled to and which switches the array between transmission and reception modes, and protects the main beamformer 20 from high energy transmit signals in the case where a microbeamformer is not used and the transducer array is operated directly by the main system beamformer. The transmission of ultrasound beams from the transducer array 6 is directed by a transducer controller 18 coupled to the microbeamformer by the T/R switch 16 and a main transmission beamformer (not shown), which can receive input from the user's operation of the user interface or control panel 38. The controller 18 can include transmission circuitry arranged to drive the transducer elements of the array 6 (either directly or via a microbeamformer) during the transmission mode.

**In** a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. **In** transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 18 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

**In** addition, upon receiving the echo signals from within the subject, it is possible to perform the inverse of the above described process **in** order to perform receive focusing. **In** other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. **In** this case, the transducer array is referred to as a phased array.

**In** case of the CMUT transducers, which require a DC bias voltage for their activation, the transducer controller 18 can be coupled to control a DC bias control 45 for the transducer array. The DC bias control 45 sets DC bias voltage(s) that are applied to the CMUT transducer elements.

For each transducer element of the transducer array, analog ultrasound signals, typically referred to as channel data, enter the system by way of the reception channel. **In** the reception channel, partially beamformed signals are produced from the channel data by the microbeamformer 12 and are then passed to a main receive beamformer 20 where the partially beamformed signals from individual patches of transducers are combined into a fully beamformed signal, referred to as radio frequency (RF) data. The beamforming performed at each stage may be carried out as described above, or may include additional functions. For example, the main beamformer 20 may have 128 channels, each of which receives a partially beamformed signal from a patch of dozens or hundreds of transducer elements. **In** this way, the signals received by thousands of transducers of a transducer array can contribute efficiently to a single beamformed signal.

The beamformed reception signals are coupled to a signal processor 22. The signal processor 22 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and microbubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. **In** Figure 1 only the receiver beamformers 12, 20 are shown, for simplicity. **In** the complete system, there will also be a transmission chain with a transmission micro beamformer, and a main transmission beamformer.

The function of the micro beamformer 12 is to provide an initial combination of signals in order to decrease the number of analog signal paths. This is typically performed in the analog domain.

The final beamforming is done in the main beamformer 20 and is typically after digitization.

The transmission and reception channels use the same transducer array 6 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 26 and a Doppler processor 28. The B mode processor 26 performs amplitude detection on the received ultrasound signal for the imaging of structures in the body, such as organ tissue and blood vessels. **In** the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 28 processes temporally distinct signals arising from tissue movement and blood flow for the detection of moving substances, such as the flow of blood cells in the image field. The Doppler processor 28 typically includes a wall filter with parameters set to pass or reject echoes returned from selected types of materials in the body.

The structural and motion signals produced by the B mode and Doppler processors are coupled to a scan converter 32 and a multi-planar reformatter 44. The scan converter 32 arranges the echo signals in the spatial relationship from which they were received in a desired image format. **In** other words, the scan converter acts to convert the RF data from a cylindrical coordinate system to a Cartesian coordinate system appropriate for displaying an ultrasound image on an image display 40. **In** the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 42 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 44, and volume renderer 42 to an image processor 30 for further enhancement, buffering and temporary storage for display on an image display 40. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 28 and tissue structure information produced by the B mode processor 26 are coupled to a quantification processor 34. The quantification processor produces measures of different flow conditions such as the volume rate of blood flow in addition to structural measurements such as the sizes of organs and gestational age. The quantification processor may receive input from the user control panel 38, such as the point in the anatomy of an image where a measurement is to be made.

Output data from the quantification processor is coupled to a graphics processor 36 for the reproduction of measurement graphics and values with the image on the display 40, and for audio output from the display device 40. The graphics processor 36 can also generate graphic overlays for display with the ultrasound images. These graphic overlays can contain standard identifying information such as patient name, date and time of the image, imaging parameters, and the like. For these purposes the graphics processor receives input from the user interface 38, such as patient name. The user interface is also coupled to the transmit controller 18 to control the generation of ultrasound signals from the transducer array 6 and hence the images produced by the transducer array and the ultrasound system. The transmit control function of the controller 18 is only one of the functions performed. The controller 18 also takes account of the mode of operation (given by the user) and the corresponding required transmitter configuration and band-pass configuration in the receiver analog to digital converter. The controller 18 can be a state machine with fixed states.

The user interface is also coupled to the multi-planar reformatter 44 for selection and control of the planes of multiple multi-planar reformatted (MPR) images which may be used to perform quantified measures in the image field of the MPR images.

The methods described herein are performed on a processing unit. Such a processing unit may be located within an ultrasound system, such as the system described above with reference to Figure 1. For example, the image processor 30 described above may perform some, or all, of the method steps detailed below. Alternatively, the processing unit may be located in any suitable system, such as a monitoring system, that is adapted to receive an input relating to a subject.

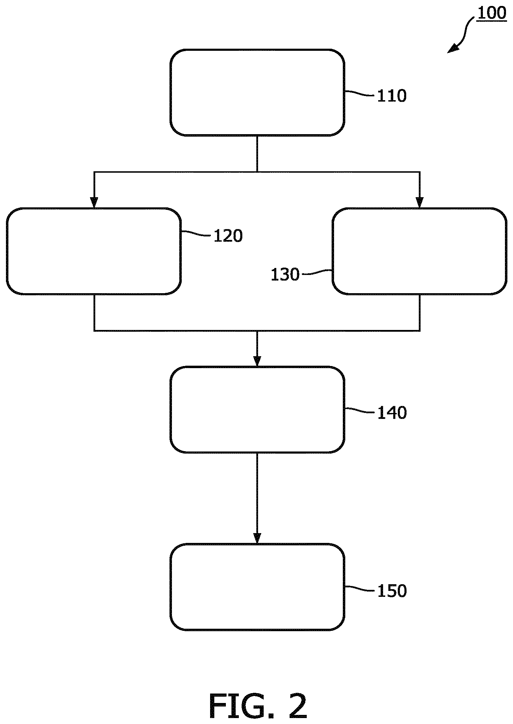

Figure 2 shows a method 100 for calculating an end-diastolic pressure-volume relationship of a subject in a non-invasive manner.

The method begins in step 110, wherein a cardiac input is obtained from a subject. The cardiac input comprises a region of interest of the subject, and in particular a left ventricle and a left atrium of a subject.

The cardiac input may, for example, include ultrasound data obtained from the subject by way of an ultrasound probe.

The ultrasound data may be obtained, for example, using a system as described above with reference to Figure 1. The ultrasound data may comprise ultrasound image data, for example B-mode ultrasound data. Further, or alternatively, the ultrasound data may include Doppler ultrasound data, such as color flow Doppler data or spectral Doppler data. **In** addition, the ultrasound data may comprise 2D ultrasound data or 3D ultrasound data.

Alternatively, the cardiac input may include a cardiac model, which simulates some, or all, of the behavior of a heart. The cardiac model may take one or more measurements from the subject in order to simulate a model of the heart. Measurements may then be taken from the simulation for use in the steps below.

The model may be a multi-scale model which represents the non-linear pressure-volume behavior of the heart.

Further, the cardiac input may include non-invasive blood pressure measurements obtained from the subject. For example, a blood pressure measurement may be obtained by way of a pressure cuff.

**In** the case where the cardiac input comprises ultrasound data, the left ventricle and the left atrium contained within the ultrasound data may be segmented.

The segmentation may be performed on the ultrasound image data or the Doppler ultrasound data. **In** other words, the ultrasound data may be partitioned into two parts, one part being the ventricular blood pool and the other the surrounding tissue. Further, the segmentation may be performed using any segmentation method suitable for identifying the left ventricle and the left atrium in the ultrasound data.

The basic structure of the heart consists of the blood filled chambers and the surrounding tissues. For the purposes of quantifying the volumes of the chambers using ultrasound image data, segmentation may refer to separating the pixels in the image into two classes, one class being the pixels from the chamber and the other class being the surrounding tissue. This segmentation may be performed using image processing methods for spatially smoothing the pixels of the image and normalizing the distribution of the greyscale values of the smoothed image. The brightness of the pixels of this processed image may then be compared to a threshold brightness. For a B-mode ultrasound image, blood samples are dark and tissue samples are bright, meaning that the two may be distinguished based on the pixel brightness.

**In** step 120, an end of diastasis volume of the left ventricle is determined based on the cardiac input. The determining of the end of diastasis volume of the left ventricle may include generating a volume segmentation of the left ventricle volume. A volume waveform may then be generated based on the segmentation of the left ventricle.

The term diastasis refers to a period during the diastolic, or filling, phase of the left ventricle. More specifically, diastasis is the period between the E- and A-waves of diastolic filling, where the initial passive filing of the ventricles has slowed, but before the atria contract to complete the active filing of the ventricles. The end of diastasis may also be referred to as the pre-A wave portion of the heartbeat cycle, the A-wave being the flow waveform resulting from the contraction of the atria.

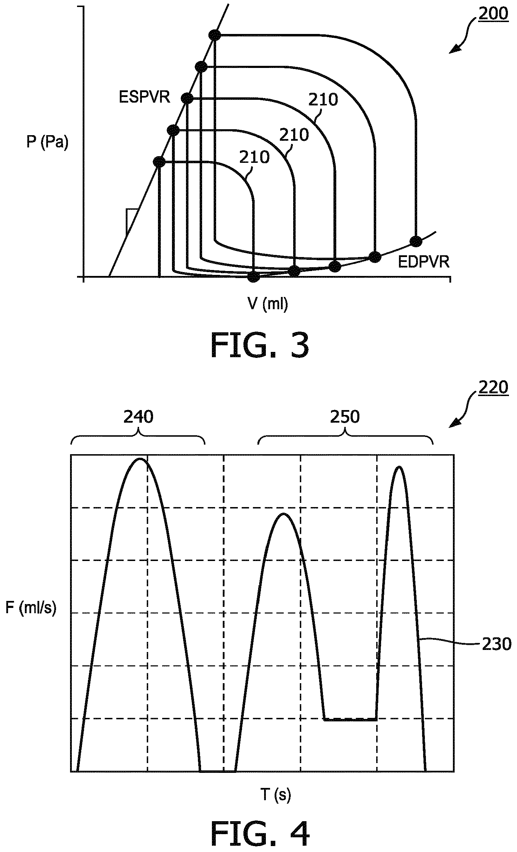

The generation of the left ventricular volume waveform may be performed by way of performing an analytical integration of an aortic flow waveform and a mitral flow waveform over time. The generation of the volume waveform is described further below with reference to Figure 4.

The fitting of the volume waveform to the segmentation of the left ventricle may, for example, be performed using a least-squares fitting. **In** other words, the measured volumes of the left ventricle, as determined by the segmentation, may be used to accurately fit the volume waveform according to the user.

**In** step 130, an end of diastasis pressure is determined in the left atrium. This determination is based on the cardiac input. **In** the example that the cardiac input includes ultrasound image data, the end of diastasis pressure in the left ventricle may be estimated based on the segmented left atrium volume.

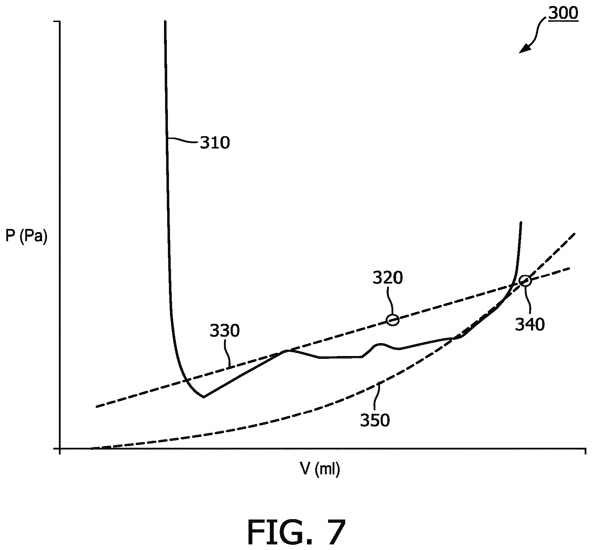

**In** step 140, a linearized ventricular pressure-volume relationship is estimated based on the end of diastasis volume of the left ventricle and the end of diastasis pressure in the left atrium. An example of a linearized ventricular pressure-volume relationship is described further below with reference to Figure 7.

In step 150, an end-diastolic pressure-volume relationship (EDPVR) is calculated based on an end-diastolic volume of the left ventricle, which may be determined from the cardiac input, and the estimated linearized ventricular pressure-volume relationship. The EDPVR may then be used to assess a passive stiffness, or other function, of the heart.

Figure 3 shows a graph 200 of pressure, P (Pa), against volume, V (ml), within the left ventricle of a subject.

The plots 210 represent pressure-volume loops within the left ventricle, which demonstrate the change in pressure and volume of the left ventricle for a range of different physiological conditions. The end systolic pressure volume relationship, ESPVR, is represented by the black circles and the EDPVR is represented by the grey circles.

Figure 4 shows a graph 220 of flow, F (ml/s), against time, T (s).

The plot 230 represents an aortic flow waveform 240 and a mitral flow waveform 250 over time, which may then be used to generate the volume waveform of the left ventricle described above.

In the example shown in Figure 4, the aortic flow waveform 240 is defined as a complete sinusoidal waveform, whereas the mitral flow waveform 250 is defined by two incomplete sinusoidal waves, thereby accounting for constant flow during diastasis. The duration and magnitude of each complete and incomplete sinusoidal waveform may be optimized numerically to yield the optimal least squares fit to the volume waveform. If Doppler ultrasound data is also available, such information can also be included to further improve the analytical waveform fit.

It should be noted that the flow waveforms are not limited to symmetric half sine waveforms, but may also be asymmetric half sine waveforms or splines, for example. Such waveforms may be incorporated into a cardiac model for use as a cardiac input.

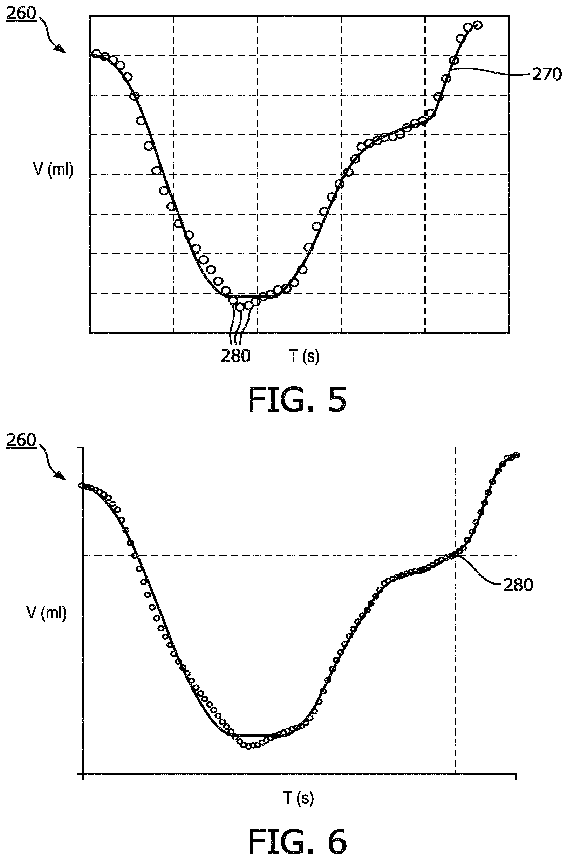

Figure 5 shows a graph 260 of volume against time for a left ventricle of a heart of the subject. In this case, the cardiac input comprises ultrasound image data, which has undergone segmentation to identify a volume of the left ventricle,
The plot 270 shows the volume waveform, as generated from the aortic flow waveform 240 and mitral flow waveform 250 of Figure 4 by way of an analytic integration. The analytical fit provides a more robust method to reconstruct a volume waveform which represents physiological events from limited frame rate ultrasound data. The volume waveform is then fit to the segmentation data 280 of the left ventricle in order to ensure the values of the volume waveform align to the actual measured volume of the subject's left ventricle.

It should be noted that, whilst only the volume waveform for the left ventricle has been shown, an equivalent volume waveform may be generated for the left atrium based on a left atrium segmentation, or for any other chamber of the heart.

Figure 6 shows the graph 260 of Figure 5 with a volume indicator 280 positioned at the end of diastasis as represented on the volume waveform. This provides a visual representation of how the end of diastasis volume of the left ventricle is determined in step 120 of the method 100 of Figure 2.

Figure 7 shows a graph 300 of pressure against volume. The graph includes a plot 310, which represents clinical data invasively obtained from a patient in order to demonstrate the accuracy of the method.

Data point 320 represents an estimation of the end of diastasis pressure. The end of diastasis pressure may be estimated based on the cardiac input in a number of ways. For example, an end of diastasis pressure may be estimated based on ultrasound data. More specifically, the end of diastasis pressure in the left ventricle may be estimated based on the segmentation of the left atrium volume from ultrasound image data, which may for example be captured using the system described with reference to Figure 1.

In other words, the volumes of the left ventricle and left atrium, which are measureable in a non-invasive manner by way of ultrasound imaging, may be used to estimate a data point 320 indicating the pressure and volume of the left ventricle at the end of diastasis.

In an example, the estimation of the end of diastasis pressure may be performed using a left atrial volume waveform using the empirical relationship described by M. Kawasaki et al., "A novel ultrasound predictor of pulmonary capillary wedge pressure assessed by the combination of left atrial volume and function: A speckle tracking echocardiography study," J. Cardiol., vol. 66, no. 3, pp. 253-262, 2015.

Plot 330 represents the linearized ventricular pressure-volume relationship as estimated in step 140 of the method 100 of Figure 2. The plot 330 represents a linear approximation of behavior of the ventricle during filling and passes through the data point 320 representing the end of diastasis pressure.

The method of Figure 2 further includes the step of determining a number of heartbeats represented in the cardiac input.

If the cardiac input consists of a single heartbeat, the pressure-volume intercept of the linearized ventricular pressure-volume relationship shown in plot 330 may be assumed to be at zero. Alternatively, it is possible to use various empirical relationships to estimate a volume at zero pressure to estimate a non-zero intercept for the linearized ventricular pressure-volume relationship, such as V_0 = 0.48 * V_ES, where V_0 is the unstressed volume and V_ES is the volume at end systole as described in Davidson et al. PLoS One. 2017; 12(4): e0176302.

If the cardiac input consists of multiple heartbeats, or new data is provided to the single heartbeat data above, a non-zero intercept may be determined for the linearized ventricular pressure-volume relationship.

The linearized ventricular pressure-volume relationship may be used to estimate the end diastolic pressure 340 at end diastolic volume.

The end diastolic volume and pressure may then be used estimate the EDPVR 350 using, for example, an empirical relationship such as the one described by S. Klotz et al., "Single-beat estimation of end-diastolic pressure-volume relationship: a novel method with potential for noninvasive application.," Am. J. Physiol. Heart Circ. Physiol., vol. 291, no. 1, pp. H403-12, 2006.

If the cardiac input consists of a single heartbeat, the EDPVR 350 may be fit with a single data point. However, if the cardiac input consists of multiple heartbeats, or new data is provided to the single heartbeat above, a least squares fitting may be performed on the EDPVR. In the example shown in Figure 7, the estimated data 340 is used to fit the EDPVR 350.

As discussed above the EDPVR may be used as an indicator of heart function. For example, it is possible to estimate the slope of the EDPVR at the current end diastolic volume. If the slope is greater than a predetermined value, such as 0.1 mmHg/ml, for example 0.2 mmHg/ml, this may indicate the presence of diastolic dysfunction.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer program comprising computer program code means which is adapted, when said computer program is run on a computer, to implement a method (100) for calculating anon-invasive end-diastolic pressure-volume relationship for a subject, the method comprising:
obtaining (110) a cardiac input representing a region of interest, wherein the region of interest comprises a left ventricle and a left atrium of a subject, wherein the cardiac input comprises ultrasound data and/or a cardiac model which takes one or more measurements from the subject to simulate the behavior of the heart;
determining (120) an end of diastasis volume of the left ventricle based on the cardiac input, wherein diastasis is a stage of diastole during a heart cycle before atrial contraction;
estimating (130) an end of diastasis pressure in the left atrium based on the cardiac input;
generating (140) a linearized ventricular pressure-volume relationship based on the end of diastasis volume of the left ventricle and the end of diastasis pressure in the left atrium; and
calculating (150) an end-diastolic pressure-volume relationship based on an end-diastolic volume of the left ventricle and the linearized ventricular pressure-volume relationship,
wherein the method (100) further comprises determining a number of heartbeats represented in the cardiac input, and, if the number of heartbeats is greater than one, the generation of the linearized ventricular pressure-volume relationship comprises fitting an intercept to the linearized ventricular pressure-volume relationship.

2. The computer program as claimed in claim 1, wherein the calculating (150) of the end-diastolic pressure-volume relationship comprises:
estimating an end-diastolic pressure at the end-diastolic volume of the left ventricle based on the linearized ventricular pressure-volume relationship; and
matching the estimated end-diastolic pressure to a generalized experimental pressure-volume relationship.

3. The computer program as claimed in any of claims 1 to 2, wherein the determining (120) of the end of diastasis volume of the left ventricle comprises generating a volume waveform of the left ventricle volume by performing an analytical integration of an aortic flow waveform and a mitral flow waveform.

4. The computer program as claimed claim 3, wherein the fitting of the volume waveform to the segmentation of the left ventricle comprises performing a least-squares fitting.

5. The computer program as claimed in any of claims 1 to 4, wherein, if the number of heartbeats is one, the generation of the linearized ventricular pressure-volume relationship comprises fitting a constant intercept to the linearized ventricular pressure-volume relationship.

6. The computer program as claimed in any of claims 1 to 4, wherein, if the number of heartbeats is one, the generation of the linearized ventricular pressure-volume relationship comprises estimating a non-zero intercept to the linearized ventricular pressure-volume relationship.

7. The computer program as claimed in any of claims 1 to 6, wherein, if the number of heartbeats is one, the calculating of the end-diastolic pressure-volume relationship comprises fitting the end-diastolic pressure-volume relationship based on a single heartbeat.

8. The computer program as claimed in any of claims 1 to 7, wherein, if the number of heartbeats is greater than one, the calculation of the end-diastolic pressure-volume relationship comprises performing a least-squares fitting of the end-diastolic pressure-volume relationship based on a plurality of heartbeats.

9. The computer program as claimed in any of claims 1 to 8, wherein the method (100) further comprises:
determining a gradient of the end-diastolic pressure-volume relationship at an end diastolic volume; and
if the gradient is greater than a predetermined threshold, generating an alert.

10. The computer program as claimed in any of claims 1 to 9, wherein the cardiac input further comprises non-invasive blood pressure measurements obtained from the subject.

11. A processing unit for calculating an end-diastolic pressure-volume relationship, wherein the processing unit is adapted to:
obtain (110) a cardiac input representing a region of interest, wherein the region of interest comprises a left ventricle and a left atrium of a subject, wherein the cardiac input comprises ultrasound data and/or a cardiac model which takes one or more measurements from the subject to simulate the behavior of the heart;
determine (120) an end of diastasis volume of the left ventricle based on the cardiac input, wherein diastasis is a stage of diastole during a heart cycle before atrial contraction;
estimate (130) an end of diastasis pressure in the left atrium based on the cardiac input;
generate (140) a linearized ventricular pressure-volume relationship based on the end of diastasis volume of the left ventricle and the end of diastasis pressure in the left atrium; and
calculate (150) an end-diastolic pressure-volume relationship based on an end-diastolic volume of the left ventricle and the linearized ventricular pressure-volume relationship,
wherein the processing unit is further adapted to determine a number of heartbeats represented in the cardiac input, and, if the number of heartbeats is greater than one, the generation of the linearized ventricular pressure-volume relationship comprises fitting an intercept to the linearized ventricular pressure-volume relationship.

12. The processing unit of claim 11, which is further adapted to:
determine a gradient of the end-diastolic pressure-volume relationship at an end diastolic volume; and
if the gradient is greater than a predetermined threshold, generate an alert.

13. An ultrasound system comprising the processing unit any of claims 11 and 12.

## Patentansprüche

1. Computerprogramm, umfassend ein Computerprogrammcodemittel, das so ausgelegt ist, dass es, wenn das Computerprogramm auf einem Computer ausgeführt wird, ein Verfahren (100) zum Berechnen einer nicht-invasiven enddiastolischen Druck-Volumen-Beziehung für einen Probanden implementiert, wobei das Verfahren Folgendes umfasst:
Erhalten (110) eines kardialen Eingangs, das einen interessierenden Bereich repräsentiert, wobei der interessierende Bereich einen linken Ventrikel und einen linken Vorhof eines Probanden umfasst, wobei der kardiale Eingang Ultraschalldaten und/oder ein Herzmodell umfasst, das eine oder mehrere Messungen am Probanden vornimmt, um das Verhalten des Herzens zu simulieren;
Bestimmen (120) eines Enddiastasevolumens des linken Ventrikels auf der Grundlage des kardialen Eingangs, wobei die Diastase ein Stadium der Diastole während eines Herzzyklus vor atrialer Kontraktion ist;
Schätzen (130) einen Druck am Ende der Diastase im linken Vorhof auf der Grundlage des kardialen Eingangs;
Erzeugen (140) einer linearisierten ventrikulären Druck-Volumen-Beziehung auf der Grundlage des Enddiastasevolumens des linken Ventrikels und des Enddiastasedrucks im linken Vorhof; und
Berechnen (150) einer enddiastolischen Druck-Volumen-Beziehung auf der Grundlage eines enddiastolischen Volumens des linken Ventrikels und der linearisierten ventrikulären Druck-Volumen-Beziehung,
wobei das Verfahren (100) ferner Bestimmen einer Anzahl von Herzschlägen umfasst, die im kardialen Eingang dargestellt sind, und, falls die Anzahl von Herzschlägen größer als eins ist, die Erzeugung der linearisierten ventrikulären Druck-Volumen-Beziehung Anpassen eines Achsenabschnitts an die linearisierte ventrikuläre Druck-Volumen-Beziehung umfasst.

2. Computerprogramm nach Anspruch 1, wobei das Berechnen (150) der enddiastolischen Druck-Volumen-Beziehung Folgendes umfasst:
Schätzen eines enddiastolischen Drucks beim enddiastolischen Volumen des linken Ventrikels auf der Grundlage der linearisierten ventrikulären Druck-Volumen-Beziehung; und
Anpassen des geschätzten enddiastolischen Drucks an eine verallgemeinerte experimentelle Druck-Volumen-Beziehung.

3. Computerprogramm nach einem der Ansprüche 1 bis 2, wobei das Bestimmen (120) des Enddiastasevolumens des linken Ventrikels Erzeugen einer Volumenwellenform des linken Ventrikelsvolumens durch Durchführen einer analytischen Integration einer Aortenflusswellenform und einer Mitralflusswellenform umfasst.

4. Computerprogramm nach Anspruch 3, wobei das Anpassen der Volumenwellenform an die Segmentierung des linken Ventrikels Durchführen einer Methode der kleinsten Quadrate umfasst.

5. Computerprogramm nach einem der Ansprüche 1 bis 4, wobei, wenn die Anzahl von Herzschlägen eins beträgt, die Erzeugung der linearisierten ventrikulären Druck-Volumen-Beziehung Anpassen eines konstanten Achsenabschnitts an die linearisierte ventrikuläre Druck-Volumen-Beziehung umfasst.

6. Computerprogramm nach einem der Ansprüche 1 bis 4, wobei, wenn die Anzahl von Herzschlägen eins beträgt, die Erzeugung der linearisierten ventrikulären Druck-Volumen-Beziehung Schätzen eines Achsenabschnitts der linearisierten ventrikulären Druck-Volumen-Beziehung ungleich null umfasst.

7. Computerprogramm nach einem der Ansprüche 1 bis 6, wobei, wenn die Anzahl von Herzschlägen eins beträgt, das Berechnen der enddiastolischen Druck-Volumen-Beziehung Anpassen der enddiastolischen Druck-Volumen-Beziehung auf der Grundlage eines einzelnen Herzschlags umfasst.

8. Computerprogramm nach einem der Ansprüche 1 bis 7, wobei, wenn die Anzahl von Herzschlägen größer als eins ist, die Berechnung der enddiastolischen Druck-Volumen-Beziehung Durchführen einer Methode der kleinsten Quadrate der enddiastolischen Druck-Volumen-Beziehung auf der Grundlage einer Vielzahl von Herzschlägen umfasst.

9. Computerprogramm nach einem der Ansprüche 1 bis 8, wobei das Verfahren (100) ferner Folgendes umfasst:
Bestimmen des Gradienten der enddiastolischen Druck-Volumen-Beziehung bei einem enddiastolischen Volumen; und
wenn der Gradient einen vorab festgelegten Schwellenwert überschreitet, Erzeugen einer Warnung.

10. Computerprogramm nach einem der Ansprüche 1 bis 9, wobei der kardiale Eingang ferner nicht-invasive Blutdruckmessungen umfasst, die vom Probanden erhalten werden.

11. Verarbeitungseinheit zum Berechnen einer enddiastolischen Druck-Volumen-Beziehung, wobei die Verarbeitungseinheit ausgelegt ist:
ein kardialer Eingang zu erhalten (110), das einen interessierenden Bereich repräsentiert, wobei der interessierende Bereich einen linken Ventrikel und einen linken Vorhof eines Probanden umfasst, wobei der kardiale Eingang Ultraschalldaten und/oder ein kardiales Modell umfasst, das eine oder mehrere Messungen am Probanden vornimmt, um das Verhalten des Herzens zu simulieren;
ein Enddiastasevolumens des linken Ventrikels auf der Grundlage des kardialen Eingangs zu bestimmen (120), wobei die Diastase ein Stadium der Diastole während eines Herzzyklus vor atrialer Kontraktion ist;
einen Druck am Ende der Diastase im linken Vorhof auf der Grundlage des kardialen Eingangs zu schätzen (130);
eine linearisierte ventrikuläre Druck-Volumen-Beziehung auf der Grundlage des Enddiastasevolumens des linken Ventrikels und des Enddiastasedrucks im linken Vorhof zu erzeugen (140); und
eine enddiastolische Druck-Volumen-Beziehung auf der Grundlage eines enddiastolischen Volumens des linken Ventrikels und der linearisierten ventrikulären Druck-Volumen-Beziehung zu berechnen (150),
wobei die Verarbeitungseinheit ferner so ausgelegt ist, dass sie eine Anzahl von Herzschlägen bestimmt, die im kardialen Eingang dargestellt sind, und, falls die Anzahl von Herzschlägen größer als eins ist, die Erzeugung der linearisierten ventrikulären Druck-Volumen-Beziehung Anpassen eines Achsenabschnitts an die linearisierte ventrikuläre Druck-Volumen-Beziehung umfasst.

12. Verarbeitungseinheit nach Anspruch 11, die ferner ausgelegt ist:
einen Gradienten der enddiastolischen Druck-Volumen-Beziehung bei einem enddiastolischen Volumen zu bestimmen; und
wenn der Gradient einen festgelegten Schwellenwert überschreitet, eine Warnung zu erzeugen.

13. Ultraschallsystem, umfassend die Verarbeitungseinheit nach einem der Ansprüche 11 und 12.

## Revendications

1. Programme informatique comprenant des moyens de code de programme informatique qui sont adaptés, lorsque ledit programme informatique est exécuté sur un ordinateur, pour mettre en œuvre un procédé (100) de calcul d'une relation pression-volume télédiastolique non invasive pour un sujet, le procédé comprenant :
l'obtention (110) d'une entrée cardiaque représentant une région d'intérêt, dans lequel la région d'intérêt comprend un ventricule gauche et une oreillette gauche d'un sujet, dans lequel l'entrée cardiaque comprend des données ultrasonores et/ou un modèle cardiaque qui effectue une ou plusieurs mesures sur le sujet pour simuler le comportement du cœur ;
la détermination (120) d'un volume de fin de diastasis du ventricule gauche sur la base de l'entrée cardiaque, dans lequel le diastasis est une phase de la diastole au cours d'un cycle cardiaque avant la contraction auriculaire ;
l'estimation (130) d'une pression de fin de diastasis dans l'oreillette gauche sur la base de l'entrée cardiaque ;
la génération (140) d'une relation pression-volume ventriculaire linéarisée sur la base du volume de fin de diastasis du ventricule gauche et de la pression de fin de diastasis dans l'oreillette gauche ; et
le calcul (150) d'une relation pression-volume télédiastolique sur la base d'un volume télédiastolique du ventricule gauche et de la relation pression-volume ventriculaire linéarisée,
dans lequel le procédé (100) comprend en outre la détermination d'un nombre de battements cardiaques représentés dans l'entrée cardiaque et, si le nombre de battements cardiaques est supérieur à un, la génération de la relation pression-volume ventriculaire linéarisée comprend l'ajustement d'une ordonnée à l'origine à la relation pression-volume ventriculaire linéarisée.

2. Programme informatique selon la revendication 1, dans lequel le calcul (150) de la relation pression-volume télédiastolique comprend :
l'estimation d'une pression télédiastolique au volume télédiastolique du ventricule gauche sur la base de la relation pression-volume ventriculaire linéarisée ; et
la mise en correspondance de la pression télédiastolique estimée avec une relation pression-volume expérimentale généralisée.

3. Programme informatique selon l'une quelconque des revendications 1 à 2, dans lequel la détermination (120) du volume de fin de diastasis du ventricule gauche comprend la génération d'une forme d'onde de volume du ventricule gauche en effectuant une intégration analytique d'une forme d'onde de flux aortique et d'une forme d'onde de flux mitral.

4. Programme informatique selon la revendication 3, dans lequel l'ajustement de la forme d'onde de volume à la segmentation du ventricule gauche comprend l'exécution d'un ajustement par les moindres carrés.

5. Programme informatique selon l'une quelconque des revendications 1 à 4, dans lequel, si le nombre de battements cardiaques est de un, la génération de la relation pression-volume ventriculaire linéarisée comprend l'ajustement d'une ordonnée à l'origine constante à la relation pression-volume ventriculaire linéarisée.

6. Programme informatique selon l'une quelconque des revendications 1 à 4, dans lequel, si le nombre de battements cardiaques est de un, la génération de la relation pression-volume ventriculaire linéarisée comprend l'estimation d'une ordonnée à l'origine non nulle pour la relation pression-volume ventriculaire linéarisée.

7. Programme informatique selon l'une quelconque des revendications 1 à 6, dans lequel, si le nombre de battements cardiaques est de un, le calcul de la relation pression-volume télédiastolique comprend l'ajustement de la relation pression-volume télédiastolique sur la base d'un seul battement cardiaque.

8. Programme informatique selon l'une quelconque des revendications 1 à 7, dans lequel, si le nombre de battements cardiaques est supérieur à un, le calcul de la relation pression-volume télédiastolique comprend l'exécution d'un ajustement par les moindres carrés de la relation pression-volume télédiastolique sur la base d'une pluralité de battements cardiaques.

9. Programme informatique selon l'une quelconque des revendications 1 à 8, dans lequel le procédé (100) comprend en outre :
la détermination d'un gradient de la relation pression-volume télédiastolique à un volume télédiastolique ; et
si le gradient est supérieur à un seuil prédéterminé, la génération d'une alerte.

10. Programme informatique selon l'une quelconque des revendications 1 à 9, dans lequel l'entrée cardiaque comprend en outre des mesures non invasives de pression artérielle obtenues auprès du sujet.

11. Unité de traitement pour le calcul de la relation pression-volume télédiastolique, dans laquelle l'unité de traitement est adaptée pour :
obtenir (110) une entrée cardiaque représentant une région d'intérêt, dans lequel la région d'intérêt comprend un ventricule gauche et une oreillette gauche d'un sujet, dans lequel l'entrée cardiaque comprend des données ultrasonores et/ou un modèle cardiaque qui effectue une ou plusieurs mesures sur le sujet pour simuler le comportement du cœur ;
déterminer (120) un volume de fin de diastasis du ventricule gauche sur la base de l'entrée cardiaque, dans lequel le diastasis est une phase de la diastole au cours d'un cycle cardiaque avant la contraction auriculaire ;
estimer (130) une pression de fin de diastasis dans l'oreillette gauche sur la base de l'entrée cardiaque ;
générer (140) une relation pression-volume ventriculaire linéarisée sur la base du volume de fin de diastasis du ventricule gauche et de la pression de fin de diastasis dans l'oreillette gauche ; et
calculer (150) une relation pression-volume télédiastolique sur la base d'un volume télédiastolique du ventricule gauche et de la relation pression-volume ventriculaire linéarisée,
dans lequel l'unité de traitement est en outre adaptée pour déterminer un nombre de battements cardiaques représentés dans l'entrée cardiaque et, si le nombre de battements cardiaques est supérieur à un, la génération de la relation pression-volume ventriculaire linéarisée comprend l'ajustement d'une ordonnée à l'origine à la relation pression-volume ventriculaire linéarisée.

12. Unité de traitement selon la revendication 11, qui est en outre adaptée pour :
déterminer un gradient de la relation pression-volume télédiastolique à un volume télédiastolique ; et
si le gradient est supérieur à un seuil prédéterminé, générer une alerte.

13. Système ultrasonore comprenant l'unité de traitement selon l'une quelconque des revendications 11 et 12.
